# EUROPEAN PATENT APPLICATION

(11) **EP 4 606 368 A1**
(43) Date of publication of application: **27.08.2025**
(21) Application number: 23879999.3
(22) Date of filing: 29.08.2023
(51) Int. Cl.: A61K 8/9789, A61Q 19/00, A61Q 19/08

(54) **METHOD FOR EXTRACTING PLANT-DERIVED EXOSOME, EXOSOME ISOLATED THEREBY, AND COSMETIC USE THEREOF**

(30) Priority: 21.10.2022 KR 20220136696
(71) Applicant: ACTIVON CO., LTD., Chungcheongbuk-do 28104 (KR); THE, DABOM CORPORATION, Seoul 03722 (KR)
(72) Inventor: JEON, Ju Hee, Cheongju-si Chungcheongbuk-do 28113 (KR); KIM, Yoon Ah, Cheongju-si Chungcheongbuk-do 28781 (KR); CHO, Se Hee, Seoul 07691 (KR); JUNG, Sung Won, Yongin-si Gyeonggi-do 16909 (KR); CHO, Youn Ki, Yongin-si Gyeonggi-do 17005 (KR); JUNG, Hyo Il, Seoul 06515 (KR); PARK, Sun Young, Seoul 03726 (KR); JEONG, Hyo Rim, Cheonan-si Chungcheongnam-do 31119 (KR); YU, Jian Ning, Seoul 03780 (KR)
(74) Representative: Vossius & Partner Patentanwälte Rechtsanwälte mbB
(86) International application number: PCT/KR2023/012746
(87) International publication number: WO 2024/085419

(57) **Abstract**

The present invention provides an exosome separation method that can increase exosome separation efficiency and obtain highly stable exosomes, and a cosmetic composition containing exosomes with improved anti-wrinkle, cell growth, anti-inflammatory, or anti-aging effects. The present invention provides a method for extracting plant-derived exosomes, the method includes (a) washing and juicing a plant to obtain a plant juice; (b) firstly centrifuging the plant juice to obtain a first supernatant; (c) secondly centrifuging the first supernatant to obtain a second supernatant; (d) subjecting the second supernatant to tangential flow filtration (TFF) to obtain a separated substance; and (e) adding antibody-attached beads to the separated substance to obtain an extract containing plant-derived exosomes. Another embodiment of the present invention provides a cosmetic composition including 0.1 to 20% by weight of plant-derived exosomes based on the total weight of the composition.

## Description

### TECHNICAL FIELD

### Cross-references to related applications.

This application claims the benefit of priority based on Korean Patent Application No. 10-2022-0136696 filed on October 21, 2022, and the entire contents of the Korean patent application are incorporated herein by reference.

The present invention relates to a method for extracting plant-derived exosome, exosome isolated thereby, and cosmetic use thereof.

### BACKGROUND ART

Exosomes refer to small vesicles with a membrane structure secreted by various cells and is defined as a type of extracellular vesicle (EVs). All cells secrete EVs to exchange information with other cells or the external environment. Exosomes range in size from approximately 50 to 200 nm and contain bioactive substances such as proteins, lipids, and nucleic acids. Exosomes exist in various cells, including those of mammals, bacteria, and plants, and reflect the state of their originating cells, so they can be used for diagnosis and treatment. Exosomes are bilayer phospholipid structures that easily penetrate cells and perform diverse physiological and pathological functions, such as immune responses and signal transduction.

Recent studies have explored the various benefits of plant-derived exosomes, revealing effects such as antioxidation and anti-inflammation. These plant-derived exosomes are natural nanoparticles that contain bioactive and signaling substances secreted by plant cells, facilitating intercellular movement and absorption, and the plant-derived exosomes are known to exhibit no toxicity compared to exosomes derived from mammals.

Due to their diverse advantages and activities, these exosomes are considered a promising material for applications in pharmaceuticals, cosmetics, and food. However, their structural characteristics as bilayer phospholipid membranes poses challenges, such as low dispersibility and a tendency to aggregate. In addition, exosomes are also unstable at high temperatures and prone to damage during the manufacturing process of cosmetic formulations, and these properties can reduce the stability of exosomes in formulations and cause precipitation. Therefore, improving the dispersibility of exosomes in aqueous solutions is necessary to increase their stability in formulations and maintain their continuous activity.

To date, in most studies, exosomes have been separated through a ultracentrifugation method based on differences in density and size from the fluid phase. However, the ultracentrifugation method has disadvantages such as the risk of breaking exosomes due to excessive shock, being labor-intensive, and time-consuming. In addition, the ultracentrifugation method has low exosome yield and cannot separate a large amount of samples at once, which lowers its industrial applicability.

Another exosome separation method is a precipitation method, where water-excluding polymers such as polyethylene glycol (PEG) bind water molecules and extract less soluble components from the solution. The precipitation method with a PEG-containing precipitation solution is easy to use and does not require special equipment, but it has the disadvantages of difficult mass production and exosome aggregation.

Meanwhile, Centella asiatica is a perennial plant belonging to the Dicotyledon class, within the Magnoliopsida subclass, specifically the Apiales order and the Apiaceae family, and has long been used as a medicinal plant. Pentacyclic triterpenes, including asiaticoside, madecasosside, asiatic acid, and madecassic acid, are known as active ingredients of Centella asiatica, and these components have been reported to have excellent effects on collagen formation in skin cells, antioxidant activity, anti-aging effects, anti-inflammatory effects, improvement of photoaging of the skin, and wound healing. In Korea, it is used as a raw material for cosmetics and wound treatment ointments.

### [Prior Art Documents]

### [Patent Documents]

1. Korean Patent Registration No. 10-2265811 (June 10, 2021)

### DISCLOSURE OF THE INVENTION

### TECHNICAL PROBLEM

The present invention is to provide a method for separating exosomes with enhanced separation efficiency and high stability.

The present invention improves the efficiency of exosome separation by further separating exosomes from extracellular vesicles using antibody-attached beads.

In addition, the present invention is to provide exosomes with excellent zeta potential.

In addition, the present invention is to provide a cosmetic composition containing exosomes with improved anti-wrinkle, anti-inflammatory, or anti-aging effects.

### TECHNICAL SOLUTION

To achieve the above object, one embodiment of the present invention provides a method for extracting plant-derived exosomes, the method comprising: (a) washing and juicing a plant to obtain a plant juice; (b) firstly centrifuging the plant juice to obtain a first supernatant; (c) secondly centrifuging the first supernatant to obtain a second supernatant; (d) subjecting the second supernatant to tangential flow filtration (TFF) to obtain a separated substance; and (e) adding antibody-attached beads to the separated substance to obtain an extract containing plant-derived exosomes.

In the present invention, the plant in step (a) may be Centella asiatica.

In the present invention, wherein in step (b), the firstly centrifuging the plant juice at 1,000 xg to 3,000 xg for 10 to 50 minutes.

In the present invention, wherein in step (c), the secondly centrifuging the first supernatant at 8,000 xg to 12,000 xg for 40 to 80 minutes.

In the present invention, the tangential flow filtration in step (d) is performed using a TFF filter with a molecular weight cutoff (MWCO) of 50,000 Da to 200,000 Da.

In the present invention, the antibody in step (e) is a plant-derived ABCG36 antibody.

In the present invention, the zeta potential of the plant-derived exosome is -30 to -15mV.

Another embodiment of the present invention provides a plant-derived exosome extracted by the method described above.

Yet another embodiment of the present invention provides a cosmetic composition comprising 0.1 to 20% by weight of the plant-derived exosome based on the total weight of the composition.

In the present invention, the cosmetic composition is for anti-wrinkle, cell growth, anti-inflammatory or anti-aging purposes.

### ADVANTAGEOUS EFFECTS

The method for separating plant-derived exosomes according to the present invention can improve the separation efficiency of exosomes by using tangential flow filtration and antibody-attached beads together.

In addition, the plant-derived exosomes according to the present invention have excellent zeta potential and are stable, and the cosmetic composition containing the same can exhibit excellent anti-wrinkle, cell growth, anti-inflammatory or anti-aging effects.

### BRIEF DESCRIPTION OF THE DRAWINGS

FIG. 1 is a diagram illustrating the results of NTA analysis and TEM analysis of Centella asiatica-derived exosomes of the embodiment.
FIG. 2 is a graph illustrating the results of NTA analysis to confirm the size distribution and number of particles of exosomes of the embodiment and Comparative Examples 1 to 3.
FIG. 3 is a diagram illustrating the zeta potential of exosomes of the embodiment and Comparative Examples 1 to 3.
FIG. 4 is a diagram confirming the cell permeability of exosomes of the embodiment and Comparative Example 5.
FIG. 5 is a result illustrating the dermal cell growth effect of exosomes of the embodiment and Centella asiatica extract of Comparative Example 4.
FIG. 6 is a result of evaluating the skin cell wound healing ability of exosomes of the embodiment and Comparative Example 5.
FIG. 7 is a result of evaluating the NO (nitric oxide) production inhibitory activity of exosomes of the embodiment and Centella asiatica extract of Comparative Example 4.
FIG. 8 is a result of evaluating the ability of exosomes of the embodiment and Centella asiatica extract of Comparative Example 4 to generate procollagen type I.
FIG. 9 is a result illustrating the temperature and weekly stability of Centella asiatica-derived exosomes of the embodiment.

### BEST MODE FOR CARRYING OUT THE INVENTION

Hereinafter, the embodiment of the present invention will be described in detail with reference to the accompanying drawings so that those skilled in the art can easily implement the present disclosure. However, the present invention can be implemented in various different forms and is not limited to the embodiments described herein.

The term "exosome" as used in the present invention collectively refers to exosomes or exosome-like extracellular vesicles having a size of about 50 to 200 nm extracted from plants.

The present invention provides a method for extracting plant-derived exosomes, the method comprising: (a) washing and juicing a plant to obtain a plant juice; (b) firstly centrifuging the plant juice to obtain a first supernatant; (c) secondly centrifuging the first supernatant to obtain a second supernatant; (d) subjecting the second supernatant to tangential flow filtration (TFF) to obtain a separated substance; and (e) adding antibody-attached beads to the separated substance to obtain an extract containing plant-derived exosomes.

In step (a), the plant may be Centella asiatica, and the Centella asiatica may be used either fresh or dried, with fresh plants being most preferred. In addition, washing may be performed using ethanol or distilled water, and plant juicing may be performed using a juicer commonly used in the art, and preferably a screw juicer may be used. The stirring speed of the screw juicer is preferably 20 to 50 rpm.

The centrifugation method of steps (b) and (c) is a separation method using the density difference between a solid and a liquid surrounding the solid, and means a method of accelerating the sedimentation of the solid using centrifugal force. Centrifugation has the advantages of being able to continuously repeat the process, being able to process a large amount in a short time, and being easy to operate in a sterile state.

The first centrifugation in step (b) is performed to obtain a first supernatant by low-speed centrifugation of the plant juice, and may be performed at 1,000 xg to 3,000 xg for 10 to 50 minutes, preferably at 1,500 xg to 2,500 xg for 20 to 40 minutes. If it is out of the above range, the separation efficiency of exosomes may be lowered, so the above range is preferable.

The second centrifugation in step (c) is performed to obtain a second supernatant by high-speed centrifugation of the first supernatant, and may be performed at 8,000xg to 12,000 xg for 40 to 80 minutes, preferably at 9,000 xg to 11,000 xg for 50 to 70 minutes. If it is out of the above range, the separation efficiency of exosomes may be lowered, so the above range is preferable.

In addition, in the present disclosure, after step (c), in order to improve exosome separation efficiency, the residue may be removed from the obtained second supernatant with a 0.2 to 0.3 µm filter under reduced pressure conditions.

The tangential flow filtration (TFF) in step (d) is a filtration method in which a solution flows in a direction perpendicular to the filtration membrane, filtering out small-sized impurities present in the solution and separating large-sized exosomes.

The rapid flow of the feed solution in TFF acts to reduce concentration polarization (product concentration at the pore surface) by 'sweeping' the membrane or hollow fiber surface. In addition, the TFF method prevents the buildup of contaminants that may clog pores. This rapid cross flow reduces pressure, which may force some of the feed solution and dissolved molecules smaller than the pores of the membrane or hollow fiber to pass through the filter. The solution that has passed through the pores is called filtrate or permeate, and molecules or particles larger than the pores remain in the feed solution and are effectively concentrated. In a preferred embodiment, the TFF of the present invention is an ultrafiltration system, and as a result of performing the TFF method as described above, the centrifuged extract may be efficiently concentrated to a volume of 1/10 to 1/100. Ultrafiltration is located in the intermediate region between microfiltration and reverse osmosis, and is a method of separating specific substances by the size difference between membrane pores and solutes. An ultrafiltration membrane may exhibit its separation performance as a molecular weight cutoff (MWCO), which is defined as the minimum molecular weight of a solute that exhibits a rejection of 90% or more by the membrane.

In step (d), tangential flow filtration may be performed using a TFF filter with a molecular weight cutoff (MWCO) of 50,000 Da to 200,000 Da, preferably a TFF filter with a molecular weight cutoff of 80,000 Da to 120,000 Da.

Step (e) is a step of obtaining an extract containing exosomes from separated substance containing exosomes separated by tangential flow filtration, which may be performed by adding antibody-attached beads, and the antibody-attached beads may be manufactured by mixing antibodies and beads in a buffer.

In the present invention, the antibody binds to exosomes or exosome-like extracellular vesicles in the separated substance to enable final separation of exosomes, and may be an antibody of a protein present in the plant extracellular vesicle membrane, and to improve the exosome extraction efficiency, it may be an ABCG36 antibody preferably, which is an antibody of a plant extracellular vesicle membrane protein, and the beads may be used without limitation as long as they are generally used as a support for antibody attachment in the art.

The antibody ABCG36 (PEN3) used in the present invention is a penetration resistance gene against external pathogens, providing non-host resistance to various pathogens on the epidermal external plasma membrane domain, and has antibacterial defense effects and serves as a defensive mechanism against external pathogens, enhancing the exosome extraction efficiency in this disclosure.

In the present invention, the amount of antibody-attached beads used may be 50 to 150 parts by weight based on 100 parts by weight of the separated substance containing exosomes, preferably 80 to 120 parts by weight, and most preferably 100 parts by weight. If the amount of antibody-attached beads used is less than 50 parts by weight, the separation efficiency of exosomes may be lowered, and if it exceeds 150 parts by weight, the exosome separation efficiency does not increase significantly compared to the added amount, which is not economical.

In the present invention, after step (e), a step of finally separating exosomes from the extract containing exosomes may be further included.

The plant-derived exosomes separated according to the present invention may have a size of 50 to 200 nm, preferably 100 to 200 nm, and may contain 5×10¹⁰ to 15×10¹⁰ particles per 1 ml of extract, preferably 8×10¹⁰ to 10×10¹⁰ particles per 1 ml of extract.

In addition, the zeta potential of plant-derived exosomes separated according to the present invention may be -30 to -15 mV, preferably -25 to -18 mV, and by exhibiting the zeta potential in the above range, it may indicate high stability in an aqueous solution.

Another embodiment of the present invention is a plant-derived exosome separated by the separation method described above, and the characteristics thereof are the same as described above, and thus will not be described below.

In addition, another embodiment of the present invention is a cosmetic composition containing 0.1 to 20% by weight of the separated plant-derived exosomes based on the total weight, and the cosmetic composition may be for anti-wrinkle, cell growth, anti-inflammatory or anti-aging.

The anti-aging effect of the present disclosure was confirmed through the growth of dermal cells, the anti-inflammatory effect was confirmed through NO production inhibitory activity, the cell growth (wound healing) effect was confirmed through the wound healing ability of skin cells, and the wrinkle improvement effect was confirmed through procollagen type I production ability.

The cosmetic composition of the present disclosure may include commonly permitted ingredients without limitation in addition to plant-derived exosomes, such as conventional adjuvants such as antioxidants, stabilizers, solubilizers, vitamins, pigments and fragrances, as well as carriers.

The cosmetic composition according to this disclosure may be manufactured into one or more formulations selected from the group consisting of solutions, topical ointments, creams, foams, nutrient cosmetics, lotions, packs, milks, makeup bases, essences, soaps, liquid cleansers, bath preparations, sunscreen creams, sunscreen oils, suspensions, emulsions, pastes, gels, lotions, powders, soaps, surfactant-containing cleansers, oils, powder foundations, emulsion foundations, wax foundations, patches, and sprays, but is not limited thereto.

In addition, the cosmetic composition in the present invention may also include one or more cosmetically acceptable carriers commonly used in general skin cosmetics, such as oils, water, surfactants, moisturizers, lower alcohols, thickeners, chelating agents, pigments, preservatives, or fragrances, but is not limited thereto. The cosmetically acceptable carriers included in the cosmetic composition of the present invention may vary depending on the formulation.

If the formulation of the present invention is an ointment, paste, cream, or gel, carrier ingredients such as animal oils, vegetable oils, waxes, paraffin, starch, tragacanth, cellulose derivatives, polyethylene glycol, silicone, bentonite, silica, talc, zinc oxide, or mixtures thereof may be used.

If the formulation of the present invention is a powder or spray, carrier ingredients such as lactose, talc, silica, aluminum hydroxide, calcium silicate, polyamide powder, or mixtures thereof may be used, and particularly in the case of a spray, propellants such as chlorofluorohydrocarbons, propane/butane, or dimethyl ether may be included.

If the formulation of the present invention is a solution or emulsion, carrier ingredients such as solvents, solubilizers, or emulsifiers may be used, including water, ethanol, isopropanol, ethyl carbonate, ethyl acetate, benzyl alcohol, benzyl benzoate, propylene glycol, or 1,3-butylene glycol oils, particularly cottonseed oil, peanut oil, corn germ oil, olive oil, castor oil, sesame oil, glycerol aliphatic esters, polyethylene glycol, or fatty acid esters of sorbitan.

If the formulation is a suspension, liquid diluents such as water, ethanol, or propylene glycol, suspending agents such as ethoxylated isostearyl alcohol, polyoxyethylene sorbitol esters, or polyoxyethylene sorbitan esters, and materials like microcrystalline cellulose, aluminum metahydroxide, bentonite, agar, or tragacanth may be used as carrier ingredients

If the formulation is soap, carrier ingredients such as alkali metal salts of fatty acids, hemiester salts of fatty acids, hydrolyzed protein salts of fatty acids, isethionate, lanolin derivatives, aliphatic alcohols, vegetable oils, glycerol, or saccharides may be used.

If the formulation is a surfactant-containing cleansing product, carrier ingredients such as alkyl sulfate, alkyl ether sulfate, sulfosuccinate monoesters, isethionate, imidazolinium derivatives, methyltaurate, sarcosinate, fatty acid amide ether sulfate, alkylamidobetaine, aliphatic alcohols, fatty acid glycerides, fatty acid diethanolamides, vegetable oils, lanolin derivatives, or ethoxylated glycerol fatty acid esters may be used.

### MODE FOR CARRYING OUT THE INVENTION

Hereinafter, the present invention will be described in more detail through specific embodiments. However, the following embodiments are only intended to illustrate the present invention, and the contents of the present invention are not limited to the following examples.

### Embodiment

1 kg of fresh Centella asiatica was washed with ethanol (90%) and distilled water, and then the remaining moisture was removed. The washed Centella asiatica was juiced using a general juicer at a low speed of 30 rpm with a screw juicer, and the obtained Centella asiatica juice was filtered through a mesh to remove suspended solids.

The Centella asiatica juice was subjected to low-speed centrifugation (2,000 xg, 30 minutes, 4°C) to remove the residue, and the first supernatant was collected, and the first supernatant was subjected to high-speed centrifugation (12,000 xg, 60 minutes, 4°C) to remove the remaining residue, and the second supernatant was collected.

The second supernatant was filtered through a 0.22 µm filter under reduced pressure conditions to finally remove the residue.

Exosomes were extracted from the second supernatant from which the residue was removed through a tangential-flow filtration (TFF) system. Specifically, exosomes were separated and purified using a tangential flow filtration system equipped with a 100,000 Da MWCO membrane.

After attaching ABCG36 antibody to beads by mixing ABCG36 antibody (ABCG36 antibody, Phytoab) and beads (7um streptavidin coated beads, Phytoab) in a buffer, add 500 µl of ABCG36 antibody attached beads to 500 µl of the separated substance separated using TFF and process for 10 minutes using a HOMM chip to obtain an extract containing exosomes. Subsequently, extracellular vesicles (EVs) were detached using Elution buffer (The DABOM) and then exosomes were finally separated.

### Comparative Example 1

1 kg of fresh Centella asiatica was washed with ethanol (90%) and distilled water, and then the remaining moisture was removed. The washed Centella asiatica was juiced using a general juicer at a low speed of 30 rpm with a screw juicer, and the obtained Centella asiatica juice was filtered through a mesh to remove suspended solids.

The Centella asiatica juice was subjected to low-speed centrifugation (2,000 xg, 30 minutes, 4°C) to remove the residue, and the first supernatant was collected, and the first supernatant was subjected to high-speed centrifugation (12,000 xg, 60 minutes, 4°C) to remove the remaining residue, and the second supernatant was collected.

The second supernatant was filtered through a 0.22 µm filter under reduced pressure conditions to finally remove the residue.

The second supernatant from which the residue was removed was subjected to ultracentrifugation (150,000 xg, 70 min, 4°C) to obtain a third supernatant and finally extract exosomes.

### Comparative Example 2

1 kg of fresh Centella asiatica was washed with ethanol (90%) and distilled water, and then the remaining moisture was removed. The washed Centella asiatica was juiced using a general juicer at a low speed of 30 rpm with a screw juicer, and the obtained Centella asiatica juice was filtered through a mesh to remove suspended solids.

The Centella asiatica juice was subjected to low-speed centrifugation (2,000 xg, 30 minutes, 4°C) to remove the residue, and the first supernatant was collected, and the first supernatant was subjected to high-speed centrifugation (12,000 xg, 60 minutes, 4°C) to remove the remaining residue, and the second supernatant was collected.

The second supernatant was filtered through a 0.22 µm filter under reduced pressure conditions to finally remove the residue.

After mixing the second supernatant with the PEG/Dextran solution, centrifugation was performed at 1,000 xg for 10 minutes at 4°C. After centrifugation, the supernatant was removed to extract exosomes.

### Comparative Example 3

1 kg of fresh Centella asiatica was washed with ethanol (90%) and distilled water, and then the remaining moisture was removed. The washed Centella asiatica was juiced using a general juicer at a low speed of 30 rpm with a screw juicer, and the obtained Centella asiatica juice was filtered through a mesh to remove suspended solids.

The Centella asiatica juice was subjected to low-speed centrifugation (2,000xg, 30 minutes, 4°C) to remove the residue, and the first supernatant was collected, and the first supernatant was subjected to high-speed centrifugation (12,000xg, 60 minutes, 4°C) to remove the remaining residue, and the second supernatant was collected.

The second supernatant was filtered through a 0.22 µm filter under reduced pressure conditions to finally remove the residue and extract exosomes.

### Comparative Example 4

Centella asiatica extract was prepared by purchasing it from the market (World Costec).

### Comparative Example 5

The Centella asiatica extract of Comparative Example 4 was subjected to low-speed centrifugation (2,000 xg, 30 minutes, 4°C) to remove the residue, and the first supernatant was collected, and the first supernatant was subjected to high-speed centrifugation (12,000 xg, 60 minutes, 4°C) to remove the remaining residue, and the second supernatant was collected.

The second supernatant was filtered through a 0.22 µm filter under reduced pressure conditions to finally remove the residue.

Exosomes were extracted from the second supernatant from which the residue was removed through a tangential-flow filtration (TFF) system. Specifically, exosomes were separated and purified using a tangential flow filtration system equipped with a 100,000 Da MWCO membrane to extract exosomes.

### Experimental Example 1

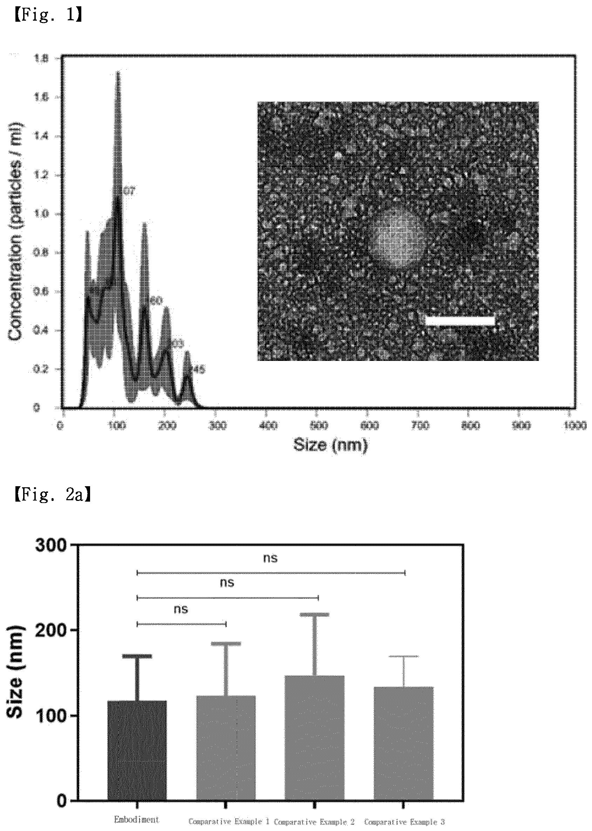

In order to confirm the particle size distribution, the number of particles per unit volume, and the shape of the exosomes extracted in the embodiment, they were analyzed by Nanoparticle Tracking Analysis (NTA) and Transmission Electron Microscope (TEM), and the results are shown in Fig. 1.

Referring to FIG. 1, the size of the exosome particles extracted according to the example averaged 100 to 200 nm, and the number per unit volume of 1 mL was found to be about 9.3 x 10¹⁰. In addition, as a result of TEM analysis, the presence of spherical particles with a lipid bilayer structure, approximately 150 nm in size was confirmed.

### Experimental Example 2

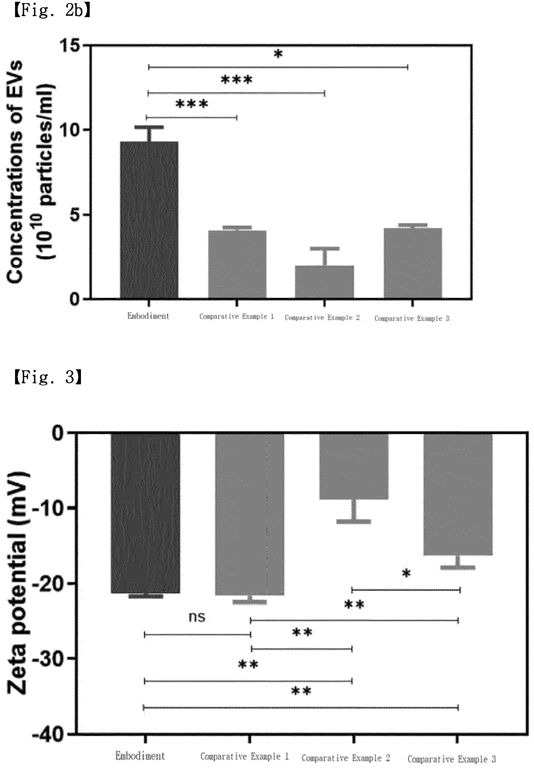

In order to confirm the particle size distribution and the number of particles per unit volume of the exosomes extracted in the embodiment and Comparative Examples 1 to 3, they were analyzed by Nanoparticle Tracking Analysis (NTA), and the results are shown in FIG. 2.

FIG. 2a is a diagram illustrating the size distribution of exosomes derived from Centella asiatica, and FIG. 2b is a diagram illustrating the number of particles per 1 mL of exosomes.

Referring to FIG. 2a, the size of the exosome particles extracted according to the embodiment and Comparative Examples 1 to 3 was similar to 100 to 200 nm.

Referring to FIG. 2b, the number per unit volume of 1 mL is about 9.3 x 10¹⁰ in the case of the embodiment, about 4.1 x 10¹⁰ in Comparative Example 1, about 2.0 x 10¹⁰ in Comparative Example 2, and about 4.2 x 10¹⁰ in Comparative Example 3. The results confirm that the largest amount of exosomes could be extracted when TFF and antibody-attached beads were used (Embodiment).

### Experimental Example 3

A colloid, which is a particle dispersed in a solution, is charged by dissociation of surface polar groups on the particle surface and adsorption of ions when present in an aqueous solution. The degree of repulsive force between particles is used as an important measure to evaluate the stability of a colloid.

In other words, if the negative (-) or positive (+) zeta potential value is large, it can be seen that the repulsive force between particles is large and the dispersion stability is good, and if it is small, it can be seen that the attractive force between particles acts greatly and the cohesive force is large. Therefore, in order to confirm the dispersion and aggregation of exosomes extracted according to the embodiment and Comparative Examples 1 to 3 in solution, zeta potential analysis was performed through Zetasizer, and the results are shown in FIG. 3.

Referring to FIG. 3, the zeta potential is -21.3 mV in the case of the example, - 21.6 mV in Comparative Example 1, -8.8 mV in Comparative Example 2, and -16.3 mV in Comparative Example 3. When beads attached (Example), it can be seen that the dispersion stability of Centella asiatica-derived exosomes extracted according to the example is the best.

### Experimental Example 4

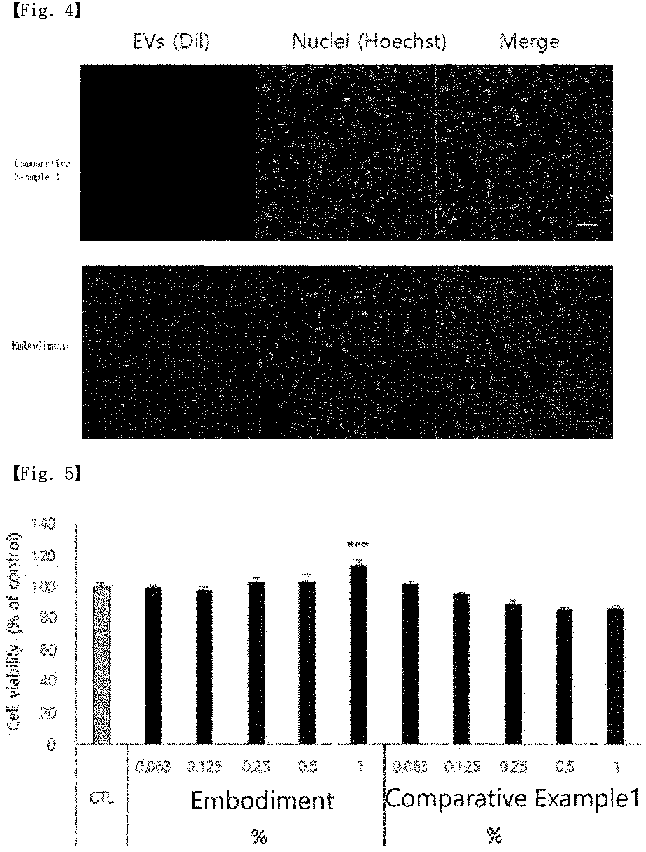

In order to measure whether the exosomes of the embodiment and Comparative Example 5 penetrate cells, the lipid layer on the surface of EV was stained using a Dil staining reagent capable of staining the lipid layer of EV, and then the unstained staining reagent was removed using a size chromatography method. After that, the same amount of EV was treated to the HaCaT cell line, and after 4 hours, the nucleus was stained using Hoechst, and then the percentage of exosomes delivered to HaCaT cells was measured. Cell proportions were measured using a Confocal LSM 880 (Zeiss), and the results are shown in FIG. 4.

Referring to FIG. 4, it can be seen that the cell permeability of exosomes extracted from Centella asiatica fresh plants (Embodiment) is superior to that of exosomes extracted from Centella asiatica extract (Comparative Example 5).

### Experimental Example 5

The dermal cell growth efficacy of the exosomes of the embodiment and the Centella asiatica extract of Comparative Example 4 was measured, and the results are shown in FIG. 5.

Human foreskin Fibroblasts (HS68) were purchased from ATCC (USA) and used. The cell culture fluid was Dulbeco's Modified Eagle's Medium (DMEM; Gibco, USA) supplemented with 10% fetal bovine serum (FBS; Gibco), 10% penicillin (100 unit/mL) and streptomycin (100 g/mL). The culture conditions were cultured in a 37°C, 95% humidity, 5% CO² incubator, and cells between passages 3 and 10 were used in the experiment.

HS68 cells, which are human dermal fibroblasts, were treated with the exosomes of the embodiment and the Centella asiatica extract of Comparative Example 4 at concentrations of 0.036, 0.125, 0.25, 0.5, and 1%, and then the cells were cultured for 24 hours, and then viability was measured using MTT of cells.

Referring to FIG. 5, in the case of Centella asiatica-derived exosomes (Embodiment), the Centella asiatica-derived exosomes (Embodiment) exhibited superior growth-promoting effects at all concentrations compared to the Centella asiatica extract (Comparative Example 4), with the highest efficacy observed at a 1% concentration.

### Experimental Example 6

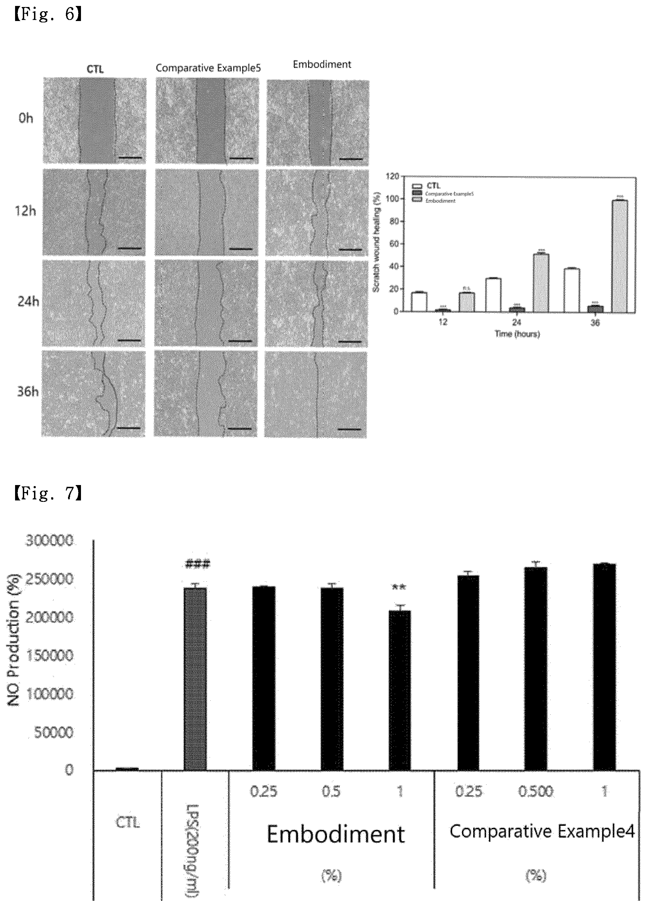

In order to confirm the wound healing effect of the exosomes of the embodiment and Comparative Example 5, wounds were inflicted on keratinocytes and then wound healing activity was confirmed, and the results thereof are shown in FIG. 6.

Specifically, 2.5×10⁵ cells/ml of human keratinocyte HaCaT cells were transferred to a 6-well plate and cultured for 24 hours in DMEM medium containing 10% (v/v) fetal bovine serum (FBS), and then a HaCaT cell monolayer was subjected to "scratch-damage" using a p200 pipette tip. The "scratch-damaged" HaCaT cell layer was treated with 1×10⁸ particles/ml of the exosomes extracted in the above embodiment and Comparative Example 5, and then further cultured for 12 hours, 24 hours, and 36 hours. Thereafter, the area ratio was calculated using the Scion-Image (Scion Corporation, MA) program.

FIG. 6 is a photograph morphologically comparing the scratched area and a graph showing the results of calculating the area ratio using the Scion-Image (Scion Corporation, MA) program. When the experimental group treated with the embodiment was treated for 24 hours and 36 hours, it can be confirmed that the "scratch damage" was healed and the scratched area was reduced compared to the negative control group (control) and Comparative Example 5, and it can be confirmed that the exosomes derived from Centella asiatica (Embodiment) had a significantly higher wound healing activity compared to the negative control group and significantly higher than the exosomes derived from Centella asiatica extract (Comparative Example 5).

### Experimental Example 7

In order to confirm the anti-inflammatory efficacy of the exosomes of the embodiment and the Centella asiatica extract of Comparative Example 4, an evaluation of the NO (Nitric oxide) production inhibition rate was conducted, and the results thereof are shown in FIG. 7.

Raw264.7 macrophages were dispensed into a 96-well plate at 1×10⁴ cells/well and cultured for 24 hours, and then treated with exosomes of the embodiment and Centella asiatica extract of Comparative Example 4 for 1 hour each. After treatment, LPS was treated at a concentration of 200 ng/ml and cultured for 24 hours to induce inflammation. Thereafter, 100 µl of cell culture supernatant was mixed with the same amount of Griess reagent, reacted in the dark at room temperature for 10 minutes, and then the absorbance was measured at 540 nm using a Spectrophotometer.

Referring to FIG. 7, when exosomes derived from Centella asiatica (Embodiment) were treated, the amount of NO production decreased in a concentration-dependent manner, and it can be seen that the NO production inhibitory activity is superior to that of Centella asiatica extract (Comparative Example 4).

### Experimental Example 8

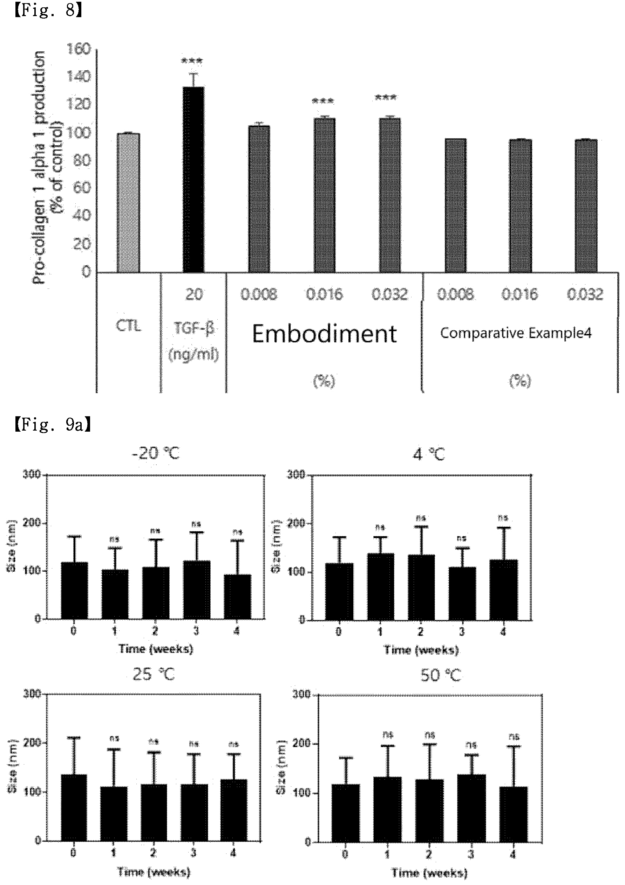

In order to confirm the wrinkle improvement efficacy of the exosomes of the embodiment and the Centella asiatica extract of Comparative Example 4, an evaluation of Procollagen Type I production ability was conducted, and the results thereof are shown in FIG. 8. Human skin fibroblasts HS68 cells were treated with DMEM medium containing 10% fetal bovine serum (FBS), penicillin 7.5 mg/L, and streptomycin 7.5 mg/L at 37°C for 24 hours under 5% CO₂. After culturing, the medium was discarded, washed with 10% PBS, and then exosomes of the embodiment and Centella asiatica extract of Comparative Example 4 were added to the new medium, respectively, and cultured for 24 hours. Thereafter, the culture solution was collected and the amount of collagen produced in the culture solution was measured using a Procollagen type-IC peptide (PIP) ELISA kit (takara). At this time, PBS was treated as a negative control group, and TGF-β 1 was treated at 5 ng/mL as a positive control group.

Referring to FIG. 8, when exosomes derived from Centella asiatica (Embodiment) were treated, the amount of collagen production increased in a concentration-dependent manner, and it can be seen that the collagen production ability is superior to that of Centella asiatica extract (Comparative Example 4).

### Experimental Example 9

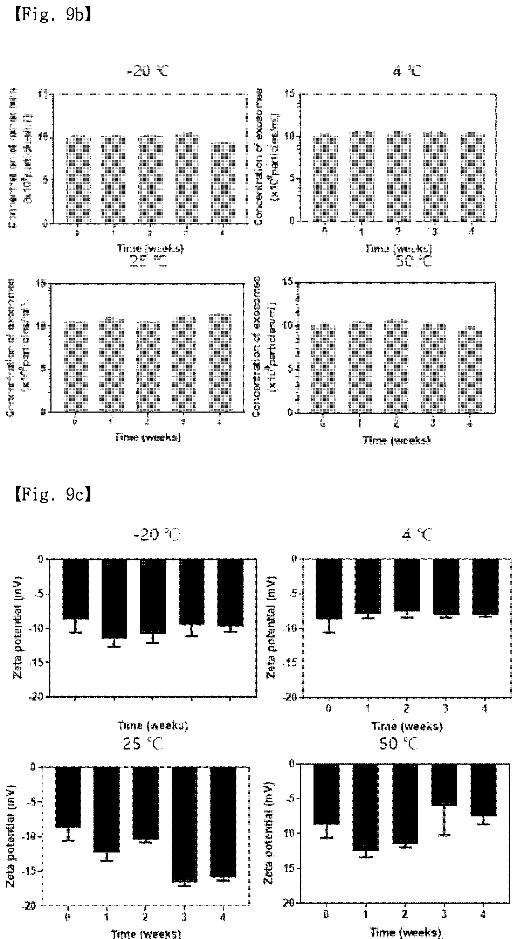

In order to confirm the stability of the exosomes extracted according to the embodiment, Centella asiatica-derived exosomes were stored at -20°C, 4°C, 25°C, and 50°C for 4 weeks, and then the particle size, concentration, and zeta potential were measured in the same manner as in Experimental Examples 1 and 2. The results thereof are shown in FIG. 9.

Referring to FIG. 9, in the case of Centella asiatica-derived exosomes (Embodiment), the size and concentration do not change significantly with temperature and time, and in the case of zeta potential, it can be seen that it is most excellent at 25°C and 3 weeks.

As described above, the plant-derived exosome extraction method according to the present invention can improve the extraction efficiency of exosomes, and accordingly, the extracted exosomes can exhibit excellent stability.

### INDUSTRIAL APPLICABILITY

The exosome separation method according to the present invention can increase the separation efficiency of exosomes and obtain highly stable exosomes, so it can be applied to cosmetics having anti-wrinkle, anti-inflammatory, or anti-aging effects.

## Claims

1. A method for extracting plant-derived exosomes, the method comprising:
(a) washing and juicing a plant to obtain a plant juice;
(b) firstly centrifuging the plant juice to obtain a first supernatant;
(c) secondly centrifuging the first supernatant to obtain a second supernatant;
(d) subjecting the second supernatant to tangential flow filtration (TFF) to obtain a separated substance; and
(e) adding antibody-attached beads to the separated substance to obtain an extract containing plant-derived exosomes.

2. The method of claim 1, wherein in step (a), the plant is Centella asiatica.

3. The method of claim 1, wherein in step (b), the firstly centrifuging the plant juice at 1,000 xg to 3,000 xg for 10 to 50 minutes.

4. The method of claim 1, wherein in step (c), the secondly centrifuging the first supernatant at 8,000 xg to 12,000 xg for 40 to 80 minutes.

5. The method of claim 1, wherein in step (d), the tangential flow filtration is performed using a TFF filter with a molecular weight cutoff (MWCO) of 50,000 Da to 200,000 Da.

6. The method of claim 1, wherein in step (e), the antibody is a plant-derived ABCG36 antibody.

7. The method of claim 1, wherein the zeta potential of the plant-derived exosomes is -30 to -15 mV.

8. A plant-derived exosome extracted by the method according to any one of claims 1 to 7.

9. A cosmetic composition comprising 0.1 to 20% by weight of the plant-derived exosomes of claim 8, based on the total weight of the composition.

10. The cosmetic composition of claim 9, wherein the cosmetic composition is for anti-wrinkle, cell growth, anti-inflammatory, or anti-aging purposes.
